# EUROPEAN PATENT APPLICATION

(11) **EP 3 855 169 A1**
(43) Date of publication of application: **28.07.2021**
(21) Application number: 19862580.8
(22) Date of filing: 19.09.2019
(51) Int. Cl.: G01N 21/85, B65G 7/08, B65G 47/248

(54) **DEVICE FOR INSPECTING ARTICLE HAVING SUBSTANTIALLY CIRCULAR SIDE SURFACE, AND DEVICE FOR TURNING ARTICLE**

(30) Priority: 21.09.2018 JP 2018176788
(71) Applicant: Miyazawa Co., Ltd., Ina-shi Nagano 399-4501 (JP)
(72) Inventor: MIYAZAWA, Yasumi, Kamiina-gun, Nagano 399-4511 (JP)
(74) Representative: Bajjon, Alexander
(86) International application number: PCT/JP2019/036746
(87) International publication number: WO 2020/059795

(57) **Abstract**

Provided is an inspection machine capable of inspecting even an article that has a substantially circular side surface. The present invention is provided with: a conveyance means for an inspection device, the conveyance means conveying an article; an identification information acquisition means that acquires identification information about a second side surface of the article, the identification information acquisition means being disposed facing the conveyance surface of the conveyance means; an imaging means that captures an image of a first side surface of the article, which is disposed on a side opposing the imaging means across the conveyance means; and a determination means that compares an image captured by the imaging means and the identification information acquired by the identification information acquisition means to determine the degree of matching between the two portions of information, a conveyance member of the conveyance means comprising a material having high transparency.

## Description

### TECHNICAL FIELD

The present invention relates to a device for inspecting an article and an article turning device.

### BACKGROUND ART

In the related art, devices for inspecting articles such as rice balls have been present. For example, Patent Literature 1 discloses an inspection device for a triangular rice ball. Image information and identification information on two types of labels attached to side surfaces of a package of a rice ball with a triangular shape that is being conveyed on a belt conveyer are read by imaging means and acquisition means disposed on both sides of a conveyance direction of an conveyance device, the rice ball is determined to be a proper product in a case in which the image information and the identification information are compared with correct information registered in advance and are the same as the registered correct information, and the rice ball is determined to be an improper product in the other cases.

### CITATION LIST

PATENT LITERATURE 1
JP-A-2018-66660

### SUMMARY OF INVENTION

### TECHNICAL PROBLEM

However, although the inspection device according to Patent Literature 1 can perform inspection on a triangular rice ball, it is difficult to appropriately dispose a round rice ball on the conveyance device such that inspection can be performed thereon even if it is attempted to inspect the round rice ball since the side surface thereof has substantially a circular shape.

Therefore, an object of the present invention is to provide an inspection machine capable of inspecting even an article having a substantially circular side surface. Another object of the present invention is to provide an article turning device that turns an article having a substantially circular side surface.

### SOLUTION TO PROBLEM

According to an aspect of the present invention, there is provided a device for inspecting an article with a substantially circular first side surface and a second side surface, the device including: conveyance means for an inspection device, the conveyance means conveying the article; identification information acquisition means that acquires identification information about the second side surface of the article, the identification information acquisition means being disposed facing a conveyance surface of the conveyance means; imaging means that captures an image of the first side surface of the article, which is disposed on a side opposing the identification information acquisition means across the conveyance means; and determination means that compares the image captured by the imaging means and the identification information acquired by the identification information acquisition means to determine matching between the two pieces of information, in which a conveyance member of the conveyance means is constituted by a material having high transparency. It is thus possible to stably convey even an article having a substantially circular side surface and to inspect the article.

The device for inspecting an article further includes: a turning device for the article, the turning device including first conveyance means that conveys the article; and turning means that turns the first side surface and the second side surface of the article. It is thus possible to extract information about the first side surface via the conveyance member constituted by the material having high transparency using the imaging means and to acquire the identification information of the second side surface directly by the identification information acquisition means with no intervention of the conveyance member constituted by the material having high transparency.

In the device for inspecting an article, the turning device is disposed upstream and downstream of the inspection device relative to a conveyance direction of the conveyance means for an inspection device. It is thus possible to acquire the identification information through conveyance with the first side surface caused to face the conveyance surface by the turning device during the inspection, and to convey the article with the second side surface caused to face the conveyance surface by the turning device after the inspection, even in a case in which it is necessary to convey the article with the second side surface caused to face the conveyance surface.

In the device for inspecting an article, the turning means includes a plurality of gripping means capable of gripping the article and second conveyance means that extends in a substantially vertical direction relative to the conveyance surface of the first conveyance means, the plurality of gripping means being disposed at intervals on the second conveyance means. It is thus possible to successively turn the article conveyed by the conveyance means.

In the device for inspecting an article, each of the gripping means includes one or more first projecting portions on an upstream side of the second conveyance means and one or more second projecting portions on a downstream side of the second conveyance means, such that the article is gripped in a pinched manner between the first and second projecting portions on the upstream side and the downstream side. It is thus possible to rotate the article while reliably gripping the article to turn the first side surface and the second side surface.

In the device for inspecting an article, the first conveyance means includes a plurality of conveyance portions that are in a substantially same plane and are separated in a vertical direction relative to a conveyance direction to dispose the gripping means between the plurality of conveyance portions. It is thus possible for the gripping means to receive the article directly from the first conveyance means.

The device for inspecting an article further includes: height position adjustment means that adjusts a position of the second conveyance means in a vertical direction relative to the conveyance surface of the first conveyance means. It is thus possible to adjust the height position of the gripping means in accordance with the size of the article.

According to another aspect of the present invention, there is provided a turning device for an article with a substantially circular first side surface and a second side surface, the device includes: first conveyance means that conveys the article; and turning means that turns the article. It is thus possible to turn the first side surface and the second side surface of the article with a substantially circular shape.

In the turning device, the turning means includes a plurality of gripping means capable of gripping the article and second conveyance means that extends in a substantially vertical direction relative to a conveyance surface of the first conveyance means, the plurality of gripping means being disposed at intervals on the second conveyance means. It is thus possible to successively turn the article conveyed by the first conveyance means.

In the turning device, each of the gripping means includes one or more first projecting portions on an upstream side of the second conveyance means and one or more second projecting portions on a downstream side of the second conveyance means, such that the article is gripped in a pinched manner between the first and second projecting portions on the upstream side and the downstream side. It is thus possible to rotate the article while reliably gripping the article to turn the first side surface and the second side surface.

In the turning device, the first conveyance means includes a plurality of conveyance portions that are in a substantially same plane and are separated in a vertical direction relative to the conveyance direction to dispose the gripping means between the plurality of conveyance portions. It is thus possible for the gripping means to receive the article directly from the second conveyance means.

The turning device further includes: height position adjustment means that adjusts a position of the second conveyance means in a vertical direction relative to the conveyance surface of the first conveyance means. It is thus possible to adjust the height position of the gripping means in accordance with the size of the article.

### ADVANTAGEOUS EFFECTS OF INVENTION

According to the present invention, it is possible to stably convey even an article having a substantially circular side surface and to inspect the article. Moreover, it is possible to easily turn the article having a substantially circular side surface.

### BRIEF DESCRIPTION OF DRAWINGS

FIG. 1 is a front view of a device for inspecting an article according to an embodiment of the present invention.
FIG. 2 is a plane view of the device for inspecting an article according to the embodiment of the present invention.
FIG. 3 is a side view of the device for inspecting an article according to the embodiment of the present invention.
FIG. 4 is a perspective view of the device for inspecting an article according to the embodiment of the present invention.
FIG. 5 is an enlarged view of imaging means and identification information acquisition means in FIG. 1.
FIG. 6 is a front view of an article turning device according to a different embodiment of the present invention.
FIG. 7 is a plane view of the article turning device according to the different embodiment of the present invention.
FIG. 8 is a side view of the article turning device according to the different embodiment of the present invention.
FIG. 9 is a perspective view of the article turning device according to the different embodiment of the present invention.
FIG. 10 is a front view when a device for inspecting an article and two article turning devices are combined according to a yet different embodiment of the present invention.
FIG. 11 is a schematic view when the article turning devices turn an article according to the different embodiment of the present invention.
FIG. 12 is a functional block diagram of a device for inspecting an article according to an embodiment of the present invention.
FIG. 13 is a functional block diagram of an article turning device according to a different embodiment of the present invention.
FIG. 14 is a flowchart illustrating an inspection method in which a device for inspecting an article and two article turning devices are combined according to a yet different embodiment of the present invention.

### DESCRIPTION OF EMBODIMENTS

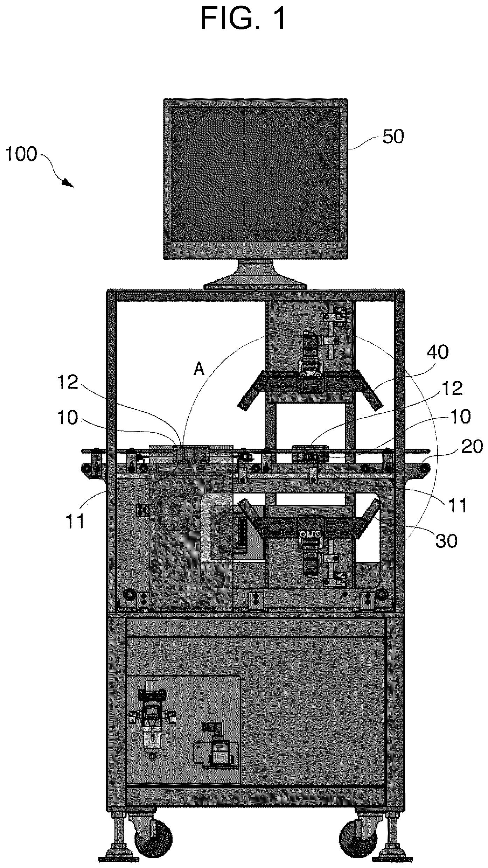

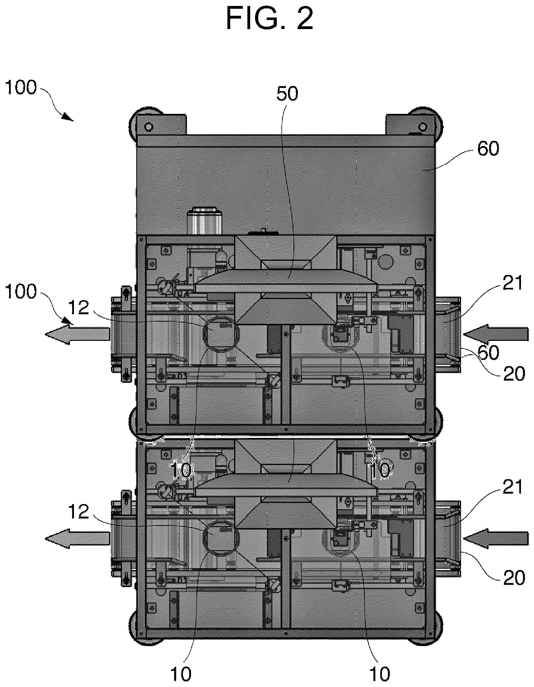

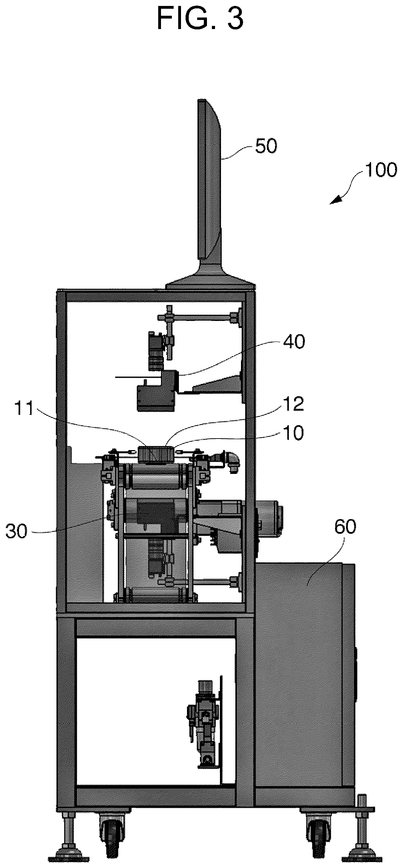

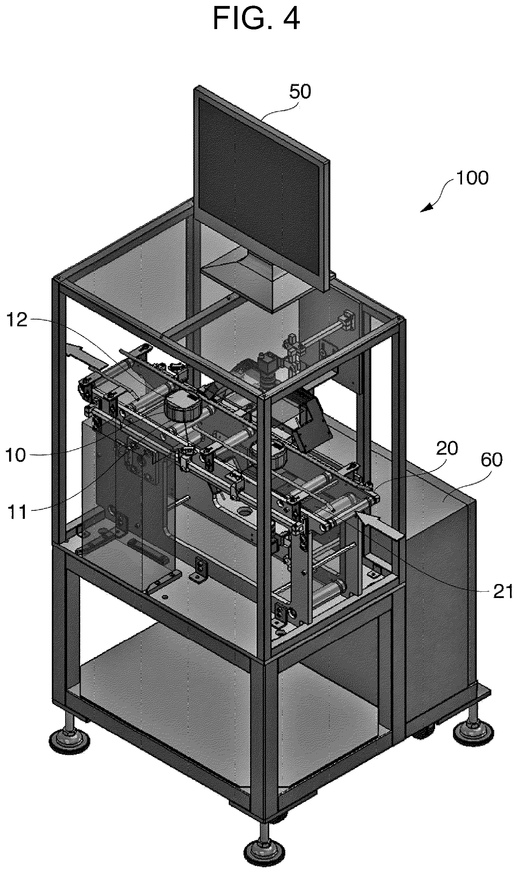

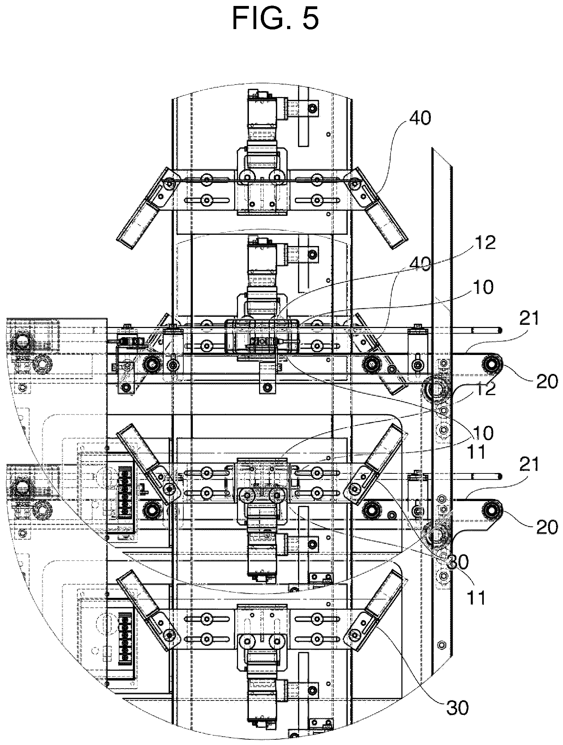

First, an inspection device 100 for an article 10 with a substantially circular first side surface 11 and a second side surface 12 according to an embodiment of the present invention will be described. Although the embodiment of the present invention includes illustrated contents, the embodiment is not limited thereto. Hereinafter, the same reference signs will be applied to common parts, and overlapping description will be omitted. Referring to FIGS. 1 to 5 and 12, there is an inspection device 100 for an article 10 with a substantially circular first side surface 11 and a second side surface 12. The article 10 is, for example, a round rice ball. The inspection device 100 includes conveyance means 20 for an inspection device that conveys the article 10, identification information acquisition means 40 that acquires identification information of the second side surface 12 of the article 10 and is disposed facing a conveyance surface 21 of the conveyance means 20 for an inspection device, and imaging means 30 that captures an image of the first side surface 11 of the article 10, which is disposed on the side opposing the identification information acquisition means 40 across the conveyance means 20 for an inspection device. The conveyance means 20 for an inspection device is, for example, a belt conveyor. The imaging means 30 is, for example, a digital camera, and the identification information acquisition means 40 is, for example, a digital camera or can be a barcode reader or a two-dimensional code reader. A label A is attached to the first side surface 11, a label B is attached to the second side surface 12, information regarding the article is described in the label A, and the label B includes a barcode, a QR code (registered trademark), or the like. In a case in which the article 10 is a round rice ball, a round rice ball wrapped with a film or the like, such as a commercially available one, is the article 10. In this case, description or the like of the information on the label A and the label B can be included in the wrapping film, and the label A and the label B can be omitted. The inspection device 100 includes determination means 60 that compares the image captured by the imaging means 30 with the identification information acquired by the identification information acquisition means 40 to determine matching between the two pieces of information. The determination means 60 is, for example, a computer, extracts the information (for example, a product name and a price) described in the label A through image processing of the captured image, compares the information with the identification information acquired by the identification information acquisition means 40 such as a barcode, a QR code (registered trademark) (two-dimensional code), or the like on the label B, and determines that the article has passed the inspection if the information and the identification information are the same, or determines that the article has failed in the inspection if the information and the identification information are different. The article is determined to have failed in the inspection in a case in which the label A or the label B have peeled off and the information cannot be read therefrom as well. Further, the conveyance surface 21 of the conveyance means 20 for an inspection device is constituted by a material having high transparency. The material having high transparency is plastic, acryl, or the like. Also, the inspection device 100 can be controlled through a touch panel 50. Since the first side surface to which the label A displaying the product name, the price, and the like has been attached is disposed on the side of the conveyance surface 21 having high transparency, it is possible to stably convey the article on the conveyance means 20 for an inspection device and to easily acquire the information described on the label A using the imaging means 30. Here, the reason that the first side surface to which the label A has been attached is disposed on the side of the conveyance surface 21 having high transparency is because in a case in which the second side surface with the label B attached thereto is disposed on the side of the conveyance surface 21 having high transparency and is imaged by the digital camera of the identification information acquisition means 40, flash may be required, and there is a concern that the flash may be reflected by the conveyance surface 21 having high transparency and the identification information cannot be acquired even if image processing is performed on the image of the barcode or the QR code (registered trademark) (two-dimensional code) captured by the identification information acquisition means 40.

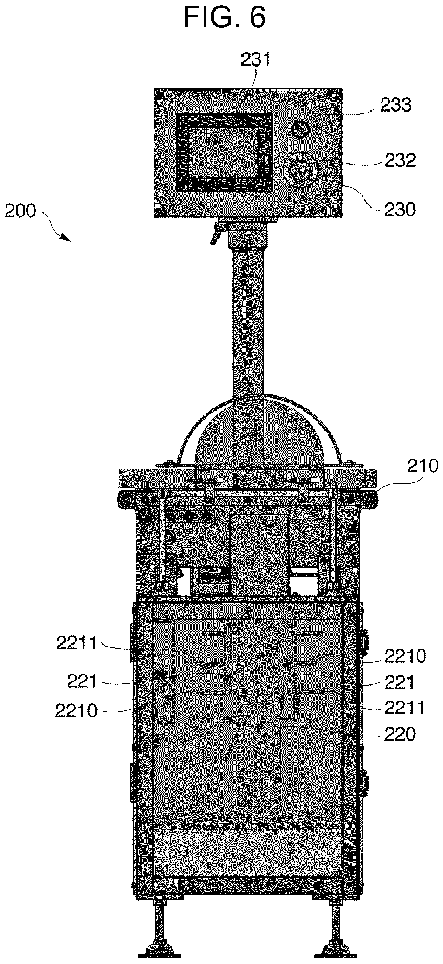

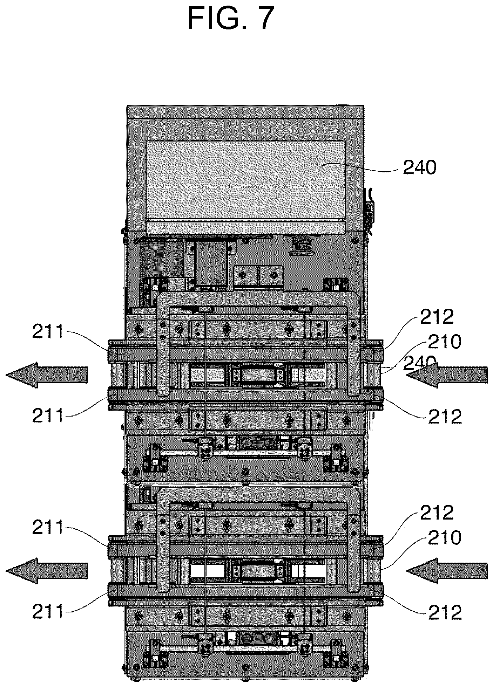

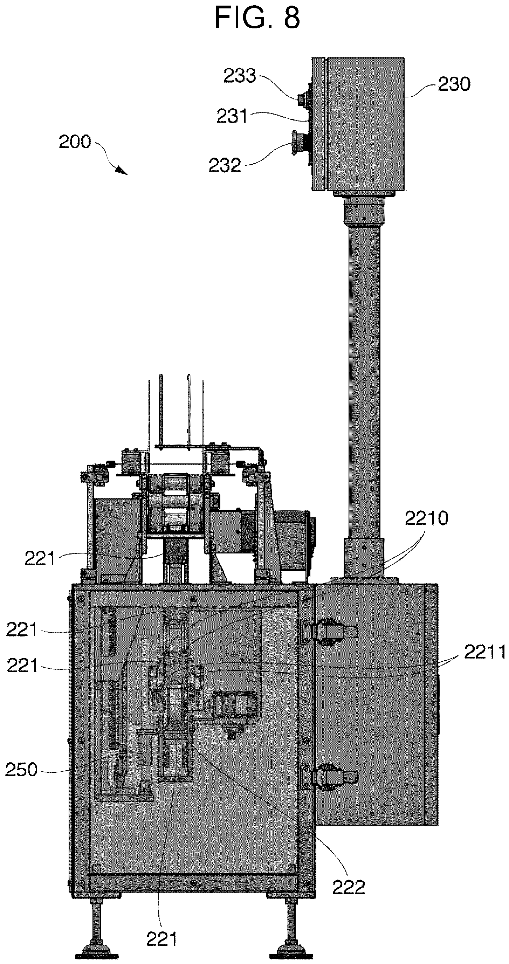

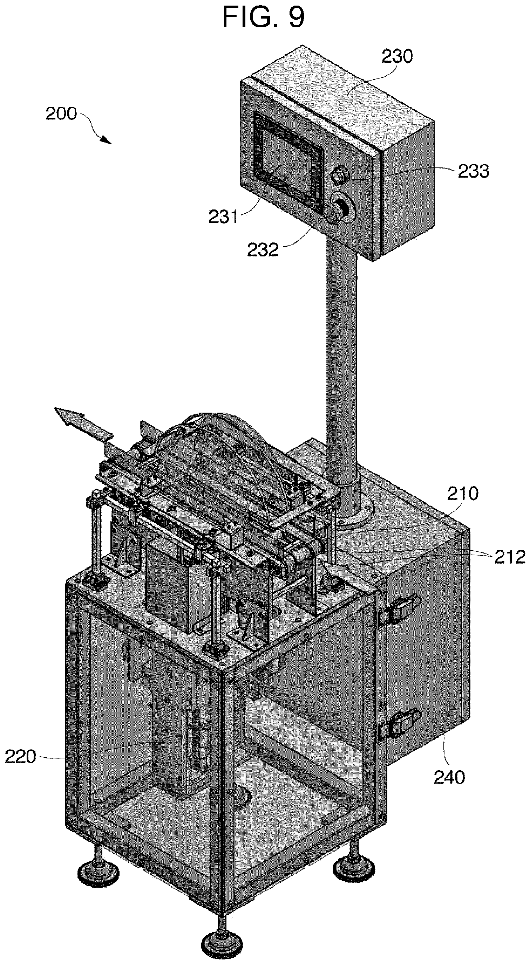

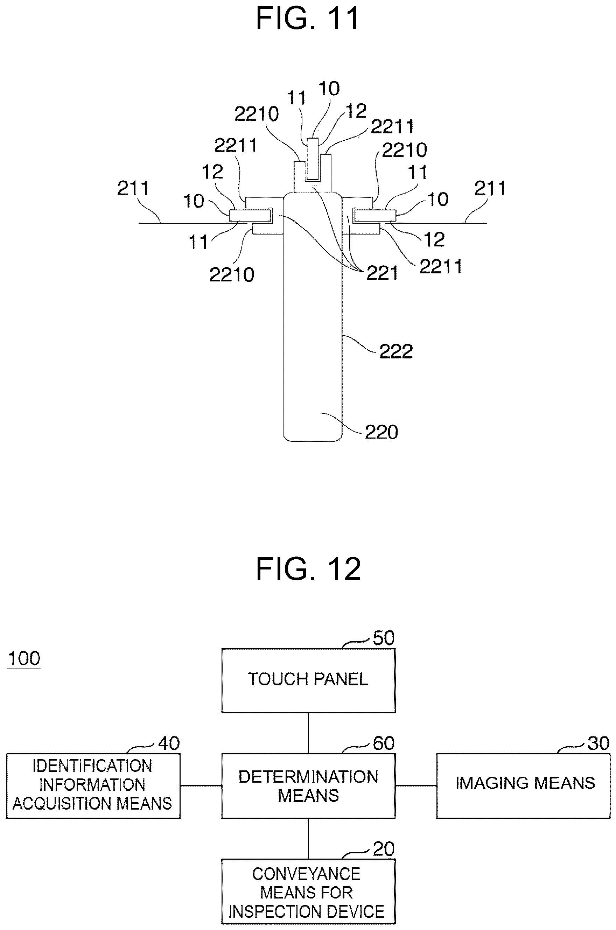

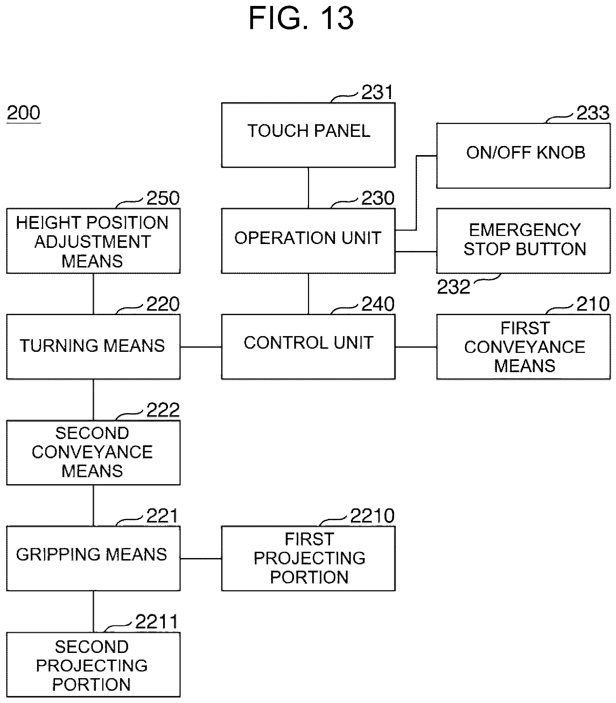

Referring to FIGS. 6 to 9 and 13, the article inspection device 100 may further include a turning device 200 for an article, which includes first conveyance means 210 that conveys the article 10 and turning means 220 that turns the first side surface 11 and the second side surface 12 of the article 10. The first conveyance means 210 is, for example, a belt conveyer. The conveyance direction of the first conveyance means 210 is the same as the conveyance direction of the conveyance means 20 for an inspection device. In a case in which the article 10 is a round rice ball, the article 10 is generally conveyed with the label B with the barcode or the QR code (registered trademark) (two-dimensional code) facing the conveyance surface 211 of the first conveyance means 210, and even if the conveyance surface 21 of the inspection device 100 is constituted by a material having high transparency, it is still difficult to image the barcode or the QR code (registered trademark) (two-dimensional code) using the digital camera of the identification information acquisition means 40 therethrough and to extract the identification information through image processing as described above. Therefore, it is possible to acquire the identification information such as the barcode, the QR code (registered trademark) (two-dimensional code), or the like on the label B on the second side surface directly by the identification acquisition means 40 with no intervention of the conveyance surface 21 constituted by a material having high transparency in the inspection device 100 by the turning means 220 turning the article 10 to cause the first side surface 11 to face the conveyance surface 211 (see FIG. 11). Also, an operation unit 230 includes a touch panel 231, an emergency stop button 232, and an ON/OFF knob 233. A control unit 240 is, for example, a computer.

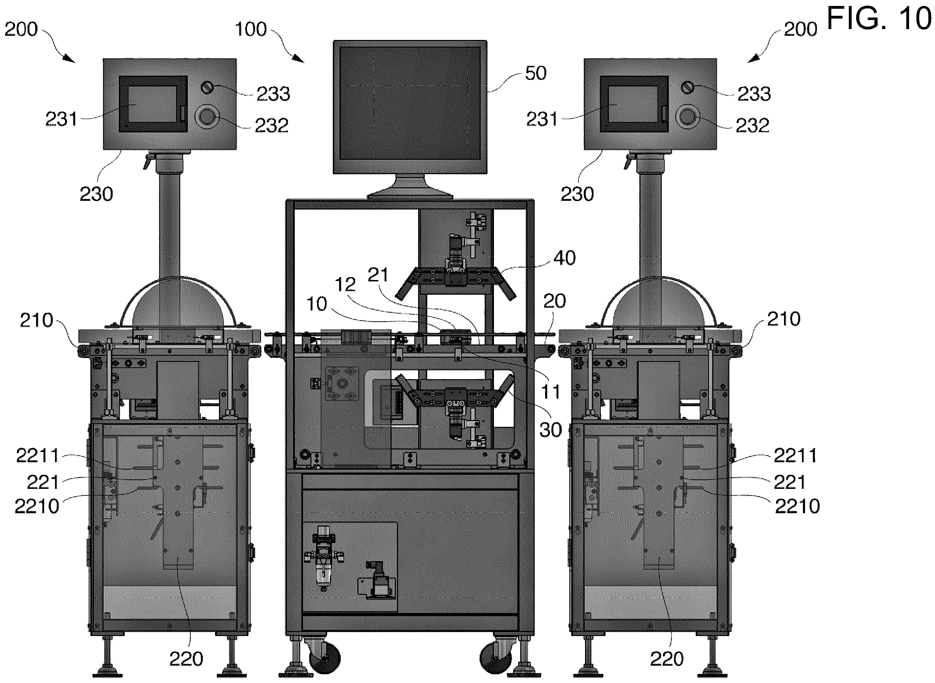

Referring to FIG. 10, the turning device 200 may be disposed at each of positions upstream and downstream of the inspection device 100 relative to the conveyance direction of the conveyance means 20 for an inspection device in the article inspection device 100. Even in a case in which it is necessary to convey the article 10 with the second side surface 12 caused to face the conveyance surface 21 as in a case in which the article 10 is a round rice ball, it is possible to acquire the identification information by conveying the article with the first side surface 11 caused to face the conveyance surface 21 by the turning device 200 during the inspection, and it is possible to convey the article with the second side surface 12 caused to face the conveyance surface 21 by the turning device 200 after the inspection. It is possible to smoothly perform conveyance and inspection of the article 10 by turning the article 10 before and after the inspection.

Returning to FIGS. 6 to 9 and 13, in the article inspection device 100, the turning means 220 of the turning device 200 may include a plurality of gripping means 221 capable of gripping the article 10 and second conveyance means 222 that extends in a substantially vertical direction relative to the conveyance surface 211 of the first conveyance means 210, and the plurality of gripping means 221 may be disposed at intervals on the second conveyance means 222. The second conveyance means 222 is, for example, a belt conveyer. The intervals of the disposition of the gripping means 221 on the conveyance means 222 is set to be the same as the intervals at which the article 10 is conveyed. Also, it is possible to successively turn the article 10 that is being conveyed by setting the conveyance speed of the first conveyance means 210 to be the same as the conveyance speed of the second conveyance means 222.

In the article inspection device 100, each of the gripping means 221 of the turning device 200 may include one or more first projecting portions 2210 on the upstream side of the second conveyance means 222 and one or more second projecting portions 2211 on the downstream side of the second conveyance means. The article 10 is gripped in a pinched manner between the projecting portions on the upstream side and the downstream side. It is thus possible to rotate the article 10 while reliably gripping the article 10 and to turn the first side surface 11 and the second side surface 12. The first projecting portions 2210 may be shorter than the second projecting portions 2211. Since the first projecting portions 2210 thus cross the conveyance surface 211 of the first conveyance means 210 first when the article 10 is passed from the first conveyance means 210 to the gripping means 221, it is possible to achieve placement on the second projecting portions 2211 without the first projecting portions 2210 colliding against the article 10 (see FIG. 11).

In the article inspection device 100, the first conveyance means 210 of the turning device 200 includes a plurality of conveyance portions 212 that are in substantially the same plane and are separated in the vertical direction relative to the conveyance direction to dispose the gripping means 221 between the plurality of conveyance portions 212. It is thus possible for the gripping means 221 to receive the article directly from the first conveyance means 210. The gripping means 221 may be disposed at a center portion of the first conveyance means 210 in the conveyance direction. It is thus possible to provide a compact turning device 200.

The article inspection device 100 may further include height position adjustment means 250 that adjusts the position of the second conveyance means 222 in the vertical direction relative to the conveyance surface 211 of the first conveyance means 210 of the turning device 200. It is thus possible to adjust the height position of the gripping means 221 in accordance with the size of the article 10. The height position adjustment means 250 may be of a hydraulic type, a pneumatic type, or the like.

In regard to a configuration (see FIG. 10) in which the inspection device 100 and the two turning devices 200 are combined according to a yet different embodiment of the present invention, an example of an operation method will be described below.

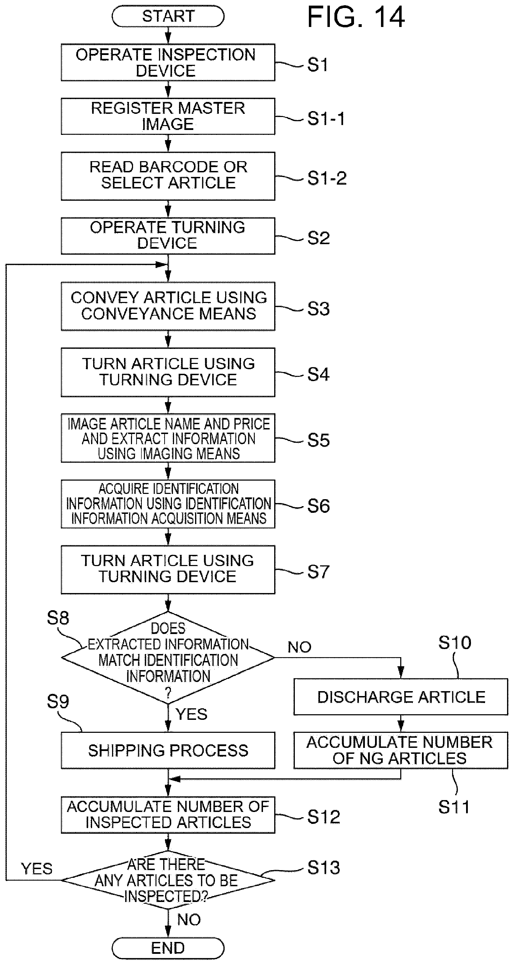

Referring to FIG. 14, the inspection device 100 is caused to operate first in Step S1. In Step S1-1, a master image of an article to be inspected is registered. At the time of the registration, it is possible to identify the article by disposing the article 10 on the conveyance means 20 for an inspection device of the inspection device 100 with the first side surface 11 facing the conveyance surface 21, extracting information such as an article name and a price on the first side surface 11 using the imaging means 30, acquiring the identification information on the second side surface 12 using the identification information acquisition means 40, and registering the information. S1-1 is repeated to register a plurality of types of articles 10 as needed. The master image is saved in a storage device of the determination means 60 in the inspection device 100. The step is skipped in a case in which no article is registered. In Step S1-2, an article to be inspected is selected. A barcode, a QR code (registered trademark) (two-dimensional code), or the like of the article to be inspected is read by such as a hand scanner, or the article to be inspected is selected using the touch panel 50. At this time, the number of articles to be inspected is input. In Step S2, the two turning devices 200 are caused to operate. In Step S3, the article 10 is conveyed by the first conveyance means 210 of the turning device 200 provided upstream of the inspection device 100. In Step S4, if the article 10 is recognized with a sensor, which is not illustrated, then the second conveyance means 222 of the turning device 200 provided upstream of the inspection device 100 operates to grip the article 10 with the first projecting portions 2210 and the second projecting portions 2211 of the gripping means 221 and turn the article. In Step S5, the article 10 is conveyed by the conveyance means 20 for an inspection device of the inspection device 100, and if the article 10 is recognized with a sensor, which is not illustrated, then the imaging means 30 images the article name, the price, and the like on the first side surface 11 of the article 10 and extracts the information through image processing. In Step S6, the identification information acquisition means 40 images the barcode or the QR code (registered trademark) (two-dimensional code) on the second side surface 12 of the article 10 and acquires the identification information through image processing. Then, in Step S7, the article 10 is conveyed by the first conveyance means 210 of the turning device 200 provided downstream of the inspection device 100. If the article 10 is recognized with a sensor, which is not illustrated, then the second conveyance means 222 of the turning device 200 provided downstream of the inspection device 100 operates to grip the article 10 with the first projecting portions 2210 and the second projecting portions 2211 of the gripping means 221 and turn the article. In Step S8, the information extracted via the imaging means 30 and the identification information acquired via the identification information acquisition means 40 are compared to determine whether or not these pieces of information match each other. Whether or not these pieces of information match the information of the master image may be determined. In a case of matching, the processing proceeds to Step S9, and the article 10 moves on to a shipping process. In a case of not matching, the processing proceeds to Step S10, and the article 10 is discharged from the conveyance surface 211 of the first conveyance means 210 of the turning device 200 through air blowing of a discharge device, which is not illustrated, without moving on to the shipping process. Note that the order of Steps S7 and S8 may be opposite, and Step S8 may be performed earlier. In this case, the article 10 for which the pieces of information do not match each other is discharged through air blowing from a conveyance surface 21 of the conveyance means 20 for an inspection device of the inspection device 100 in Step S10, and the article 10 for which the pieces of information match each other is turned by the turning device 200 in Step S7. In a case in which the pieces of information do not match each other in Step S10, the number of articles that are determined to be NG is accumulated, and the accumulation information is saved in the storage device of the determination means 60 in the inspection device 100 in Step S11. In Step S12, the number of inspected articles 10 is accumulated, and this is also saved in the storage device of the determination means 60 in the inspection device 100. In Step S13, whether or not any articles 10 to be inspected are left from the accumulated number of the inspected articles, and Steps S3 to S13 described above are repeated if any articles 10 to be inspected are left, or the inspection of the articles 10 ends if no articles 10 to be inspected are left. Note that the inspection can be continued regardless of the number of inspected articles without inputting the number of articles to be inspected in Step S1-2.

The device as a combination of the inspection device 100 and the two turning devices 200 may be disposed in a process of a line for shipping the article 10, and a control device of the line may perform central management of operations. The control device is an information terminal, examples of which include a computer, a tablet terminal, and a smartphone. The control device includes a CPU, a storage device, a removable media drive, a communication device, an input device, an output device, and an interface, which are not illustrated. The CPU executes a computer program installed in the control device and controls the storage device and the like. The storage device stores the computer program and stores information generated and to be used by the CPU. The removable media drive is used when the computer program is installed in the control device from a storage medium with the computer program stored therein. The communication device is used when the computer program is downloaded/installed in the control device from another computer via a communication line network. The input device outputs information generated by user's operations to the CPU. The output device outputs the information generated by the CPU in such a manner that the user can recognize the information. The interface outputs, to the CPU, information generated by external equipment connected to the control device and outputs information generated by the CPU to the external equipment. The external equipment includes the inspection device 100 and the turning devices 200. Examples of the storage device include a ROM, a RAM, an HDD, and a non-volatile memory. Examples of the removable media drive include a CD-ROM drive and a DVD drive. Examples of the input device include a keyboard and a mouse. Examples of the output device include a display and a printer.

The computer program installed in the control device is formed of a plurality of computer programs to cause the control device to realize each of a plurality of functions. The plurality of functions include operations of the inspection device 100 and the turning devices 200. Note that the functions and the like of the control device of the line as described above can also be included in the determination means 60 of the inspection device 100 and the control unit 240 of each turning device 200 as needed.

Although examples of the present invention have been described above in detail with reference to the drawings, specific configurations are not limited to the examples, and design modifications that do not depart from the gist of the present invention are included in the present invention.

### REFERENCE SIGNS LIST

10 Article
11 First side surface
12 Second side surface
20 Conveyance means for inspection device
30 Imaging means
40 Identification information acquisition means
50 Touch panel
60 Determination means
100 Inspection device
200 Turning device
210 First conveyance means
220 Turning means
221 Gripping means
222 Second conveyance means
230 Operation unit
231 Touch panel
232 Emergency stop button
233 ON/OFF knob
240 Control unit
250 Height position adjustment means
2210 First projecting portion
2211 Second projecting portion

## Claims

1. A device for inspecting an article with a substantially circular first side surface and a second side surface, the device comprises:
conveyance means for an inspection device, the conveyance means conveys the article;
identification information acquisition means acquiring identification information about the second side surface of the article, the identification information acquisition means is disposed facing a conveyance surface of the conveyance means;
imaging means capturing an image of the first side surface of the article, the imaging means is disposed on a side opposing the identification information acquisition means across the conveyance means; and
determination means comparing the image captured by the imaging means and the identification information acquired by the identification information acquisition means to determine matching between the two pieces of information,
wherein a conveyance member of the conveyance means is constituted by a material having high transparency.

2. The device for inspecting an article according to claim 1, further comprises:
a turning device for the article, the turning device includes first conveyance means conveying the article, and
turning means turning the first side surface and the second side surface of the article.

3. The device for inspecting an article according to claim 2, wherein the turning device is disposed upstream and downstream of the inspection device relative to a conveyance direction of the conveyance means for an inspection device.

4. The device for inspecting an article according to claim 2 or 3, wherein the turning means includes a plurality of gripping means capable of gripping the article and second conveyance means extending in a substantially vertical direction relative to the conveyance surface of the first conveyance means, the plurality of gripping means is disposed at intervals on the second conveyance means.

5. The device for inspecting an article according to claim 4, wherein each of the gripping means includes one or more first projecting portions on an upstream side of the second conveyance means and one or more second projecting portions on a downstream side of the second conveyance means, such that the article is gripped in a pinched manner between the first and second projecting portions on the upstream side and the downstream side.

6. The device for inspecting an article according to claim 4 or 5, wherein the first conveyance means includes a plurality of conveyance portions being in a substantially same planar and being separated in a vertical direction relative to a conveyance direction to dispose the gripping means between the plurality of conveyance portions.

7. The device for inspecting an article according to any one of claims 4 to 6, further comprises:
height position adjustment means that adjusts a position of the second conveyance means in a vertical direction relative to the conveyance surface of the first conveyance means.

8. An article turning device for an article with a substantially circular first side surface and a second side surface, the article turning device comprises:
first conveyance means conveying the article; and
turning means turning the article.

9. The article turning device according to claim 8, wherein the turning means includes a plurality of gripping means capable of gripping the article and second conveyance means extending in a substantially vertical direction relative to a conveyance surface of the first conveyance means, the plurality of gripping means are disposed at intervals on the second conveyance means.

10. The article turning device according to claim 9, wherein each of the gripping means includes one or more first projecting portions on an upstream side of the second conveyance means and one or more second projecting portions on a downstream side of the second conveyance means, such that the article is gripped in a pinched manner between the first and second projecting portions on the upstream side and the downstream side.

11. The article turning device according to claim 9 or 10, wherein the first conveyance means includes a plurality of conveyance portions being in a substantially same planar and being separated in a vertical direction relative to the conveyance direction to dispose the gripping means between the plurality of conveyance portions.

12. The article turning device according to any one of claims 9 to 11, further comprises:
height position adjustment means adjusting a position of the second conveyance means in a vertical direction relative to the conveyance surface of the first conveyance means.
